# EUROPEAN PATENT APPLICATION

(11) **EP 4 238 550 A1**
(43) Date of publication of application: **06.09.2023**
(21) Application number: 21886070.8
(22) Date of filing: 21.10.2021
(51) Int. Cl.: A61K 8/81, A61K 8/06, A61Q 17/04, A61K 8/92, A61K 8/31, A61K 8/36, A61K 8/85

(54) **METHOD FOR PRODUCING COMPOSITION**

(30) Priority: 29.10.2020 JP 2020181404
(71) Applicant: Japan Coating Resin Corporation, Osaka-shi, Osaka, 541-0044 (JP)
(72) Inventor: ONISHI Keisuke, Hirakata-shi, Osaka 573-1132 (JP); HATAKEYAMA Eri, Hirakata-shi, Osaka 573-1132 (JP)
(74) Representative: Grünecker Patent- und Rechtsanwälte PartG mbB
(86) International application number: PCT/JP2021/038986
(87) International publication number: WO 2022/091952

(57) **Abstract**

In a method for producing a composition containing (A) polyvinyl alcohol, (B) an oil-soluble UV absorber, (C) a solid oil, and (D) water, said (B), (C) and (D) are emulsified in the presence of said (A) at a temperature above the melting point of said (C).

## Description

### TECHNICAL FIELD

This invention relates to a method for producing a composition, especially for UV-protective cosmetics.

### BACKGROUND ART

Sunburn is caused by sunlight, especially ultraviolet rays, and results in browning of the skin, loss of skin elasticity, and wrinkles. In order to prevent these problems, cosmetics containing UV absorbers are used.

Among UV absorbers, oil-soluble UV absorbers have excellent UV absorbing effects, but if they are added as is into cosmetics, irritation and stickiness may occur, posing a problem in terms of comfortableness of use.

To address this problem, various cosmetic products have been studied. For example, in Patent Document 1, a UV-protective cosmetic containing an ionic surfactant, an oil-soluble UV absorber, a solid oil, and water is known. This cosmetic is manufactured by mixing and emulsifying an ionic surfactant, an oil-soluble UV absorber, a solid-oil, and water, cooling, and then mixing with a water-soluble polymer such as polyvinyl alcohol.

### PRIOR ART DOCUMENT

### PATENT DOCUMENT

Patent Document 1: Japanese Patent Publication 2017-7969

### SUMMARY OF THE INVENTION

### PROBLEM TO BE SOLVED

However, the UV-protective cosmetic obtained in the above-identified Patent Document 1 had problems in terms of salt miscibility and storage stability.

Therefore, it is an object of this invention to obtain compositions, especially cosmetics, with excellent salt miscibility and storage stability, even when an oil-soluble UV absorber is contained.

### MEANS FOR ACHIEVING THE OBJECT.

This invention solved the above-described problem by using (A) polyvinyl alcohol, (B) oil-soluble UV absorbent, (C) solid oil, and (D) water, and emulsifying them under predetermined conditions.

In particular, the gist of the present invention lies in the following [1] to [3].
[1] A method for producing a composition containing (A) polyvinyl alcohol, (B) an oil-soluble UV absorber, (C) a solid oil, and (D) water, characterized in that said (B), (C) and (D) are emulsified in the presence of said (A) at a temperature above the melting point of said (C).
[2] The method for producing the composition according to [1], characterized in that said composition is used in a UV protective cosmetic.

### Effect of the Invention

Since the present invention uses the predetermined manufacturing method, the resulting composition has a core-shell structure in which the oil-soluble UV absorber is incorporated into the particle center and the solid oil is phase-separated near the water phase, forming a core-shell structure with the oil-soluble UV absorber as the core portion and the solid oil as the shell portion, whereby the composition has excellent salt miscibility and storage stability.

### EMBODIMENTS OF THE INVENTION

The following is a detailed description of a method for manufacturing a composition of the present invention.

This invention relates to a method for producing an oil-soluble UV absorber-containing composition (hereinafter simply referred to as the "composition") containing a polyvinyl alcohol (Component (A)), an oil-soluble UV absorber (Component (B)), a solid oil (Component (C)), and water (Component (D)).

Water as Component (D) may be, for example, pure water or ion-exchanged water.

### <Raw materials used>

### [Component (A): Polyvinyl alcohol]

The above-described polyvinyl alcohol (PVA) can be obtained by a known method, i.e., saponification of polyvinyl acetate obtained by polymerizing vinyl acetate. The polyvinyl alcohol used in the composition of the present invention is not particularly limited, but preferably has a saponification degree of 70% or more, and especially preferably 80% or more. The upper limit of the degree of saponification is not limited and is allowed up to 100%, but generally 90% or less is particularly suitable. A degree of polymerization of 100 or more is preferred. The upper limit of the degree of polymerization is about 4000, which is the upper limit of commercial products currently offered.

Commercial products of PVA include the GOHSENOL series made by Mitsubishi Chemical Corp. (EG-03P, EG-05P, EG-18P, EG-22P, EG-30P, EG-40P, EG-48P, N-300, NL-05, AL-06R, GH-23, GH-22, GH-20R, GH-17R, GM-14R, GL-05, GL-03, KH-20, KH-17, KL-05, KL-03, NK-05R, etc.), and the KURARAY Poval series made by Kuraray Co. Ltd., the DENKA Poval series made by Denka Corporation, the SHIN-ETSU Poval series made by Shin-Etsu Chemical Industry Co., Ltd., the UNITIKA Poval series made by Unitika, which all match the above properties.

As the specially modified PVA, the GOHSENX series made by Mitsubishi Chemical Corp. (Z-100, Z-200, Z-205, Z-210, Z-220, Z-300, Z-320, Z-410, K-434, L-3266, CKS-50, T-330H, T-330, T-350), etc., may be used alone or in combination.

The amount of the PVA in the composition of the present invention is preferably equal to or more than 0.1% by weight of the composition, and particularly preferably equal to or more than 0.3% by weight. Also, this amount is preferably equal to or less than 10% by weight, and particularly preferably less than or equal to 5% by weight. If this amount is less than 0.1% by weight of the composition, a suspension envisaged in the present invention cannot be obtained, and if higher than 10% by weight, the viscosity will be too high as to cause uncomfortableness to use.

### [Component (B): Oil-soluble UV absorber]

The above-mentioned oil-soluble UV absorber (hereinafter sometimes simply referred to as the "UV absorber") refers to a UV absorber whose solubility in water is 5% or less by weight.

The following commercial products can be listed as oil-soluble UV absorbers approved for cosmetic applications. In the following, they are described according to the "INCI nomenclature".

PABA, homosalate (HMS), benzophenone-3 (BENZ-3), butyl methoxydibenzoylmethane (BMDBM), octocrylene (OC), polyacrylamidomethyl benzylidene camphor, ethylhexyl methoxycinnamate (EMC, OMC), isoamyl p-methoxycinnamate (IMC), ethylhexyltriazone (OT, ET), drometrizole trisiloxane, diethylhexyl butamide triazone (DBT), 4 methylbenzylidene camphor (MBC), 3-benzylidene camphor (BC), ethylhexyl salicylate (OS, ES), ethylhexyl dimethyl PABA (OD-PABA, ED-PABA), benzophenone-4 (BENZ-4), methylene bisbenzotriazolyl tetramethylbutylphenol (bisoctyltriazole, BOT), bisethylhexyloxyphenol methoxyphenyltriazine (AT), poly silicon 15 or diethylamino hydroxybenzoyl hexyl benzoate, 1-(4-tert-butylphenyl)-3-(4-methoxyphenyl)propane-1,3-dione, 4-isopropyl dibenzoylmethane, 2-hydroxy-4 methoxybenzophenone, 2,4,6-tris(biphenyl)-1,3,5-triazine (TBT), methanone 1,1'-(1,4-piperazinediyl)bis[1-[2-[4-(diethylamino)-2-hydroxybenzoyl]phenyl]] (CAS No. 919803-06-8), 1,1-di(carboxy-(2',2'-dimethylpropyl))-4,4-diphenylbutadiene, bis-ethylhexyloxyphenol methoxyphenyl triazine, merocyanine derivatives or benzylidene malonate, t-butyl methoxydibenzoylmethane (CAS No. number 70356-09-1) and the like. A compound thereof or a mixture thereof can be used as the UV absorber.

Particularly preferred are octocrylene, ethylhexyl methoxycinnamate, isoamyl p-methoxycinnamate, octyl methoxycinnamate, as well as mixtures of the respective isomers, which are isopentyl 4-methoxysilicate, homosalate, salicylic acid octyl, 1-(4-tert-butylphenyl)-3-(4-methoxyphenyl)propane-1,3-dione, 4-isopropyl dibenzoylmethane, 2-hydroxy-4-methoxybenzophenone, and bis-ethyl hexyloxyphenol methoxyphenyl triazine, and mixtures of these UV absorbers.

In the method for manufacturing the composition of this invention described below, when the solid oil (Component (C)) solidifies after emulsification at high temperature, a core-shell particle is formed in which the UV absorber forms the core and the solid oil forms the shell with phase separation, so that a UV absorber which is liquid at 20°C should be selected. If the UV absorber is solid at 20°C, phase separation does not occur during cooling after emulsification, and the UV absorber is uniformly dispersed within the particle.

Considering that the UV absorber does not contact the skin when the composition is used, it is preferable that the UV absorber is liquid at the temperature of use. If the UV absorber is solid, it can be dissolved in an oil or the like to give it fluidity.

Here, the oil is preferably fluid at 20°. The kind of the oil is not particularly limited as long as it is selected from those used in ordinary cosmetics. For example, the following can be used: straight or branched-chain hydrocarbon oils such as liquid paraffin, light isoparaffin, squalane, squalene, etc.; ester oils such as fatty acid esters including neopentyl glycol dicaprate, isopropyl palmitate, and alkyl benzoate, and polyhydric alcohol fatty acid esters such as pentaerythritol tetra-2-ethylh; silicone oils such as dimethylpolysiloxane, dimethylcyclopolysiloxane, methylphenylpolysiloxane, methyl hydrogen polysiloxane, and higher alcohol-modified silicone oil; and fluorine oils such as fluoropolyether and perfluoroalkyl ether silicone. Also, the oil may be selected from natural oils such as vegetable oils including jojoba oil and olive oil; and natural oils such as animal oils including liquid lanolin.

Among these, straight or branched-chain hydrocarbon oils, ester oils, silicone oils, etc. are preferred as liquid oils other than the oil-soluble UV absorbers from the viewpoint of reducing stickiness and improving compatibility with oil-soluble UV absorbers. One of these oils may be used alone or a plurality of them may be used in combination.

The weight ratio of the UV absorber to the entire core-shell particle (encapsulation ratio) can be expressed as a content ratio, and 20% or more by weight is preferable, and 30% or more by weight is even more preferable. Also, 75% by weight or less is preferable, and 70% by weight or less is even more preferable. If this ratio is less than 20% by weight, the UV absorption capacity tends to be insufficient. On the other hand, if more than 75% by weight, the thickness of the shell becomes thinner and the stability of the particle tends to decrease.

The UV absorber content in the composition of the present invention is preferably 3% by weight or more, and more preferably 5% by weight or more, from the viewpoint of stability of the suspension and for stably carrying the UV absorber. Also, 40% by weight or less is preferable and 30% by weight or less is more preferable. If the content of the UV absorber is less than 3% by weight, the UV absorbing capacity tends to be insufficient, while if more than 40% by weight, the concentration of the entire composition becomes higher and the frequency of contact between particles increases, so that the viscosity of the composition decreases due to particle agglomeration and other factors, and the stability of the composition tends to decrease.

### [Component (C): solid oil].

Component (C), or solid oil, is an oil that is solid at 25°C. Such solid oils are not particularly limited and include, for example, vegetable waxes such as candelilla wax (melting point: 66-75°C), rice wax (melting point: 77-86°C), sunflower wax (melting point: 65-80°C), carnauba wax (melting point: 80-86°C), and wood wax (melting point: 50-56°C); animal waxes such as beeswax (melting point: 62-65°C) and whale wax (melting point: 42-52°C); mineral waxes such as montan wax (82-95°C) and ozokerite (melting point: 66-78°C); petroleum waxes such as microcrystalline wax (melting point: 60-90°C), paraffin (40-70°C), and sericin (60-80°C); synthetic waxes such as hardened castor oil (melting point: 85°C), hydrogenated jojoba oil (melting point: 66-70°C), 12-hydroxystearic acid (melting point: 75-78°C), stearic acid amide (melting point: 101°C), silicone wax (melting point: 28-80°C), and polyethylene wax (melting point: 94-152°C); fatty acids such as lauric acid (melting point: 42-44°C), myristic acid (melting point: 52-54°C), palmitic acid (melting point: 60-63°C), stearic acid (melting point: 67-70°C), and behenic acid (melting point: 75-79°C); higher alcohols such as myristyl alcohol (melting point: 33-45°C), cetanol (melting point: 46-55°C), cetiaryl alcohol (melting point: 46-55°C); stearyl alcohol (melting point: 54-62°C), and behenyl alcohol (melting point: 65-72°C); and polyester resins such as polycaprolactone (melting point: 40-60°C).

Among these, a solid oil with a melting point of 60 to 80°C is preferable as Component (C) from the viewpoint of obtaining a stable suspension.

Any Component (B) that corresponds to Component (C) is regarded as Component (B). Compounds that fall under this category include, for example, t-butyl methoxydibenzoylmethane (CAS No. 70356-09-1) (melting point: 155°C).

As Component (C), from the viewpoint of improving the stability of the suspension, the total amount of the fatty acids with a carbon number of 20 or less (hereinafter sometimes referred to as "C20 or less") is preferably 3% by weight or more of the total amount of Component (C), and more preferably 5% by weight or more of the total amount of Component (C). Component (C)may be, for example, the above-above-described paraffin or ceresin. The above-mentioned fatty acids of C20 or less meet the requirements of Component (C), i.e., they are fatty acids that are in a solid state at 25°C.

On the other hand, if the content of at least one of a branched hydrocarbon such as isoparaffin and a saturated cyclic hydrocarbon such as cycloparaffin contained in Component (C) is 10% or more by weight of the total amount of Component (C), then, as Component (C), the total amount of the fatty acids of C20 or less may be less than 3% by weight of the total amount of Component (C), in terms of improving the stability of the suspension.

These may vary depending on how much "branched hydrocarbon (isoparaffin) or saturated cyclic hydrocarbon (cycloparaffin)" is contained in that component. Although the reason is not clear, it is thought that when there is a large amount of isoparaffin or cycloparaffin in the hydrocarbon, the crystals that form when changing from a liquid to a solid at high temperatures are small and do not impair particle stability, while in normal paraffin, which contain a smaller amount of isoparaffin or cycloparaffin, because the crystals produced when changing from a liquid to a solid state are large, the crystals tend to destabilize the particle.

For example, some microcrystalline waxes contain 17% by weight of isoparaffin and 24% by weight of cycloparaffin, etc., and such waxes achieve the object of the present invention even without the presence of fatty acids of C20 or less. However, even in such cases, the total amount of fatty acids of C20 or less may be 3% or more by weight, and preferably 5% or more by weight, of the total amount of Component (C).

The amount of the isoparaffin or the cycloparaffin can be measured by mass spectrometry.

Candelilla wax contains 15.0% fatty acid of C20 or less, rice wax contains 85.5% fatty acid of C20 or less, sunflower wax contains 0% fatty acid of C20 or less, Carnauba wax contains 8.7% fatty acid of C20 or less, beeswax contains 94.7% or more fatty acid (100% European or 94.7% oriental). This is based on the description in the Japan Oil Chemistry Association (1990) "Ester Composition of Wax," Oil and Fat Chemistry Handbook (Revised 3rd ed., 133-137).

The above-described vegetable waxes, animal waxes, etc. are mixtures of fatty acids and/or higher alcohols, while the petroleum waxes are hydrocarbons, and, because fatty acids, higher alcohols, and hydrocarbons have a distribution of carbon numbers, emulsion stability could be insufficient. Emulsification stability can be improved by using fatty acids of C20 or less mentioned above such that the total amount of the fatty acids of C20 or less in combination with other fatty acids in Component (C) will preferably be 3% by weight or more. The upper limit is preferably 80% by weight, and more preferably 70% by weight. Stearic acid and lauric acid are preferable as the fatty acids of C20 or less.

One of the above-described Components (C) may be used alone or a plurality of them may be used in combination, and the content of Component(s) (C) in the composition is, from the viewpoint of stability of the suspension and to stably carry the UV absorber, preferably 3% by weight or more, and more preferably 5% by weight or more. Also, 40% or less by weight is preferable, and 30% or less by weight is more preferable.

### [Optional Component.]

As an optional component used in combination with the above-described(A) polyvinyl alcohol, a water-soluble polymer can be used during emulsification to improve the stability of the suspension. Such water-soluble polymers are not limited, and any natural, semi-synthetic, or synthetic polymers can be used.

Examples of the natural polymers include xanthan gum, carrageenan, algin, microfibrillated cellulose acid, etc.

Examples of the semi-synthetic polymers include semi-synthetic polysaccharide polymers, specifically, modified polysaccharides such as hydroxycellulose, hydroxypropyl cellulose, sodium carboxymethyl cellulose, methyl cellulose, hydroxymethyl cellulose, cationized cellulose, sucrose fatty acid esters, etc.

Examples of the synthetic polymers include acrylic polymers including acrylic acid polymers such as carbomer (cross-linked polyacrylic acid), polyacrylic acid, sodium polyacrylate, acrylic acid/sodium polyacrylate alkyl copolymer, polyacrylamide, (sodium acrylate/sodium acryloyldimethyltaurate) copolymer, (hydroxyethyl acrylate/sodium acryloyldimethyltaurate) copolymer, (acrylamide/ammonium acrylate) copolymer, and polyacrylate-13; polyvinylpyrrolidone, cationized polyvinylpyrrolidone, etc.

Among these, polyvinylpyrrolidone and sucrose fatty acid esters are preferred.

### [Method of producing the composition.]

The composition according to the present invention can be produced by emulsifying Components (B), (C) and (D) in the presence of Component (A) and, if necessary, said water-soluble polymer, at a temperature not less than the melting point of Component (C).

Since Components (B), (C), and (D) are emulsified at a high temperature of not less than the melting point of Component (C) in the presence of, for example, Component (A), as described above, when Component (C) (solid oil) solidifies, Component (B)is incorporated within Component (C) such that Component (B) (UV absorber) and Component (C) (solid oil) are separated into two phases with Component (B) forming a core and Component (C) forming a shell, thus forming a core-shell particle encapsulating the UV absorber in the solid oil.

In the composition of this invention, since Component (B) is incorporated within Component (C), Component (B) is not in contact with the outside. Therefore, it is possible to obtain a UV absorber-encapsulated composition in which deterioration of the feeling of use such as irritation and stickiness can be suppressed, and salt miscibility and storage stability are good.

As the emulsification method described above, known methods such as dispersion by mechanical shear force such as homogenizers, microfluidizers, ultrasonic emulsifiers, high-pressure emulsifiers (high-pressure homogenizers), creamixes, and double motion; membrane emulsification methods; and microchannel emulsification methods can be employed.

The temperature conditions during emulsification are not limited as long as they are not less than the melting point of the solid oil used, but it is preferable that the temperature is between 70 and 95° C.

When a dispersion containing the above-mentioned essential components or, if desired, also the above-mentioned optional components, is emulsified, it is possible to further reduce the particle size of the droplets by, if the dispersion is emulsified under high pressure, setting the pressure at 1 to 100 MPa, preferably 2 to 70 MPa, more preferably about 3 to 50 MPa, and if by ultrasonic waves, setting the output to 10 to 200 W/h, 30 to 180 W/h, more preferably 50 to 150 W/h.

After the emulsification process, it is desirable to cool the emulsion not rapidly but at a cooling rate of 0.5 to 3°C/min, for phase separation between the UV absorber and the solid oil. Insufficient stirring during the rapid cooling could result in adhesion and solidification of the particles at the contact area with the container and the particles becoming unstable.

The average particle diameter of the core-shell particles thus obtained may be determined according to the desired average particle diameter of the particles, and is usually around 0.2 to 20 pm, preferably 0.4 to 10 pm. By setting the amounts of the solid oil, the UV absorber, and a dispersion stabilizer, a non-volatile content, and the temperature during emulsification, etc. within the above-described ranges as appropriate, a droplet average particle diameter in the above-described range can be obtained.

### [Uses.]

The composition of the present invention forms a core-shell structure with the oil-soluble UV absorber as the core portion and the solid oil as the shell portion, and thus have excellent salt miscibility and excellent storage stability. Therefore, the composition can be advantageously used in UV-protective cosmetics.

### EXAMPLE

The present invention will be described in more detail with the following examples, but the invention is not limited to the following examples as long as they do not exceed the gist thereof. First, the evaluation method and raw materials are shown below.

In the examples, "part" and "%" are on the weight basis unless otherwise noted.

### <Evaluation Method>

### [Emulsion stability]

Suspensions obtained were filtered through a 150-mesh filter cloth, and the state of the suspensions were observed after 10 minutes, and evaluated according to the following criteria.
⊚ : The suspension could be filtered without clogging the filter cloth, and no oil floating or other layer separation was observed.
○ : Some residue remained on the filter cloth. However, no oil floating or other layer separation was observed in the suspension.
X : The suspension did not pass through the filter cloth at all. Or, oil floating was observed in the suspension.

]

### [Average particle size measurement]

The average particle size in terms of volume was measured using a laser diffraction/scattering particle size distribution analyzer LA-950V2 (made by HORIBA, Ltd.).

### [salt miscibility].

Calcium chloride (CaCl₂, made by Fujifilm Wako Pure Chemicals Co., Ltd.) was adjusted to a prescribed concentration in ion-exchanged water. 1 ml of the suspension was placed in an aluminum case with a top diameter x bottom diameter x height H (mm) of 75 × 35 × 20. Approximately 0.1 ml of the calcium chloride aqueous solution was dropped into the case, and then mixed with a spatula to check for agglomerates and evaluated according to the following criteria.
○ : No agglomerates were observed.
X : Agglomerates were generated.

### [Storage stability].

20 g was weighed into a 30 ml polyethylene container, and the viscosity was measured using a vibration viscometer VISCOMATE VM- 100A (made by SECONIK). After the measurement, the polyethylene container was placed in an oven at 50°C. After a predetermined period of time, the container was removed and allowed to cool, then stirred slowly with a spatula and the viscosity was measured in the same manner. The viscosity was measured at a temperature range of 24±2°C.

The sample solution was, after 4 weeks in the 50°C oven, evaluated according to the following criteria.
⊚ : The rate of change in viscosity after 4 weeks was less than 20%.
○ : The rate of viscosity change after 4 weeks was not less than 20% and less than 60%.
△ : The rate of viscosity change after 4 weeks was 60% or more, and the sample solution had fluidity after 4 weeks.
X : After 4 weeks, the sample solution had no fluidity due to gelatinization, etc. Thus, the viscosity was unmeasurable.

### <Raw materials>

### Component (A): Polyvinyl alcohol

### •GOHSENOL EG-05, made by Mitsubishi Chemical Co., Ltd. (hereinafter referred to as "EG05")

### [Component (B): Oil-soluble UV absorber]

### •Ubinal MC80, made by BASF Corporation (hereinafter referred to as "MC80")

### [Component (C): Solid oil]

- Candelilla wax: Refined candelilla wax TYPE No. 1, made by Nippon Wax Co. Melting point: 68-75°C, fatty acid content rate of C20 or less: 15.0% by weight
- Beeswax: SR BEESWAX-PA-JP, made by Iwase Kospha Co., Ltd.; melting point: 62 to 65°C; fatty acid content rate of C20 or less: 95% by weight
- Carnauba wax: Carnauba WAX No. 3, made by S. Kato & CO.; melting point: 80 to 86°C, fatty acid content rate of C20 or less: 8.7% by weight
- Paraffin: paraffin made by Kanto Chemical Co., Ltd.; melting point: 60-62°C; contains no fatty acids of C20 or less
- Microcrystalline wax: MULTIWAX W-445, made by Shima Trading Co., Ltd.; melting point: 60-85°C; contains no fatty acids of C20 or less
- Polycaprolactam: Capa 2201, made by Perstorp, melting point: 40-50°C; contains no fatty acids of C20 or less
   Stearic acid: Lunac S-98, made by Kao Corporation, melting point: 68-70°C Lauric acid: Lunac L-98, made by Kao Corporation, melting point: 42-44°C

### [Optional components]

- Polyvinylpyrrolidone: Polyvinylpyrrolidone K90, made by Fujifilm Wako Pure Chemicals Corporation; 2% aqueous solution (hereinafter referred to as "PVP")
- Cellulose nanofiber: Exilva F 01-L, made by Boregard, 2% aqueous solution (hereinafter referred to as "F01L")
- Sucrose laurate: L-1695, made by Mitsubishi Chemical Corporation (hereinafter referred to as "L-1695")

### [Others]

- Anionic surfactant: Sodium lauryl sulfate, Emer-O: made by Kao Corporation (hereinafter referred to as "lauryl sulfate Na")
- Oleic acid: Lunac O-V, made by Kao Corporation; melting point: 13.4°C; contains no fatty acids of C20 or less.

### (Example 1)

A 10% aqueous solution of EG05 heated and dissolved in ion-exchanged water was prepared in advance. 12 parts by weight of the 10% EG05 aqueous solution, 21 parts by weight of MC80, 17 parts by weight of candelilla wax, and 50 parts by weight of ion-exchanged water were measured into a 300 ml stainless steel container and soaked in a 90°C hot bath for 30 minutes or more, to dissolve the Candelilla wax. After dissolving the Candelilla wax, a suspension was prepared by emulsifying the wax using an ultrasonic processing device (Ultara Generator Model US-300T, made by NIHONSEIKI KAISHA LTD.; output of approx. 100 W/h, 3 min). Then, the suspension was slowly cooled while stirring at room temperature. The suspension thus obtained was subjected to the above-described measurements and evaluations. The results are shown in Table 1.

### (Example 2)

A suspension was obtained in the same manner as in Example 1, except that the amount of the 10% EG05 aqueous solution was changed to 6 parts by weight and the amount of ion-exchanged water was changed to 56 parts by weight. The suspension obtained was subjected to the above-described measurements and evaluations. The results are shown in Table 1.

### (Reference Example 1)

A suspension was obtained in the same manner as in Example 1, except that 1.2 parts by weight of sodium (Na) lauryl sulfate was used instead of the 10% EG05 aqueous solution, and the content of the ion-exchanged water was changed to 60.8 parts by weight. The suspension obtained was subjected to the above-described measurements and evaluations. The results are shown in Table 1.

### (Comparative Example 1)

To 100 weight parts of the suspension obtained in the reference example, 12 weight parts of the 10% EG05 aqueous solution was added, and the suspension was stirred and mixed at room temperature. The suspension obtained was subjected to the above-described measurements and evaluations. The results are shown in Table 1.

### (Comparative Example 2)

A suspension was obtained in the same manner as in Comparative Example 1, except that the amount of the 10% EG05 aqueous solution was changed to 6 parts by weight. The suspension obtained was subjected to the above-described measurements and evaluations. The results are shown in Table 1.

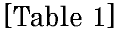

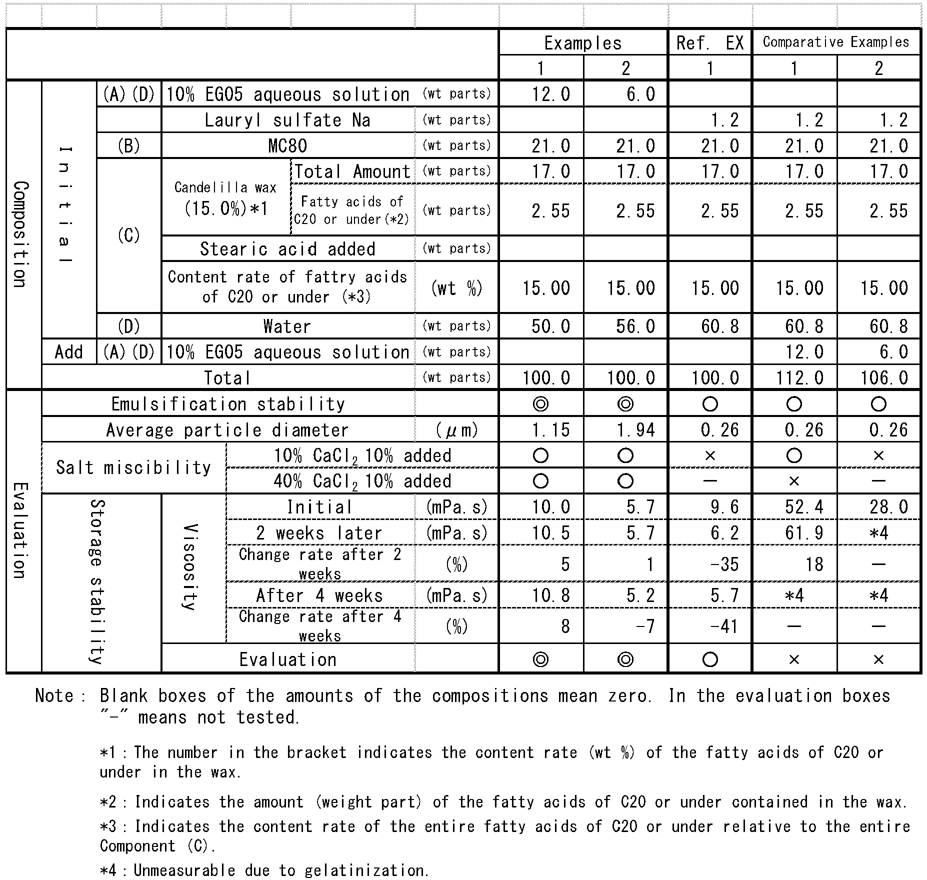

### (Results)

By adopting the configuration of the present invention, Example 1 showed good salt miscibility with no agglomerates generated when 10% of a 40% aqueous solution of calcium chloride (CaCl₂) was added.
It also exhibited good storage stability due to its small rate of change in viscosity, which was 5% from the initial stage to 2 weeks later and 8% from the initial stage to 4 weeks later.

On the other hand, in Comparative Example 1, (A) polyvinyl alcohol was not used during emulsification, but was added after emulsification, so that when 10% of a 40% aqueous solution of calcium chloride (CaCl₂) was added, aggregates were generated and salt miscibility deteriorated. The rate of change in viscosity between the initial stage and 2 weeks later was about 18%, but the viscosity after 4 weeks was unmeasurable, indicating that the storage stability was also poor.

In Comparative Example 2, even when a 10% aqueous solution of calcium chloride (CaCl₂) was added by 10%, agglomerates were generated, the initial viscosity was high, and the viscosity after 2 weeks was unmeasurable. In other words, both salt miscibility and storage stability were poor.

### (Examples 3-19)

Suspensions were obtained in the same manner as in Example 1, except that the elements used and their amounts are as described in Table 2. The suspensions were subjected to the above-described measurements and evaluations. The results are shown in Table 2.

### (Comparative Example 3)

A suspension was obtained in the same manner as in Example 1, except that a non-solid oil (oleic acid) was used instead of Component (C). The suspension obtained was subjected to the above-described measurements and evaluations. The results are shown in Table 2.

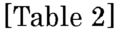

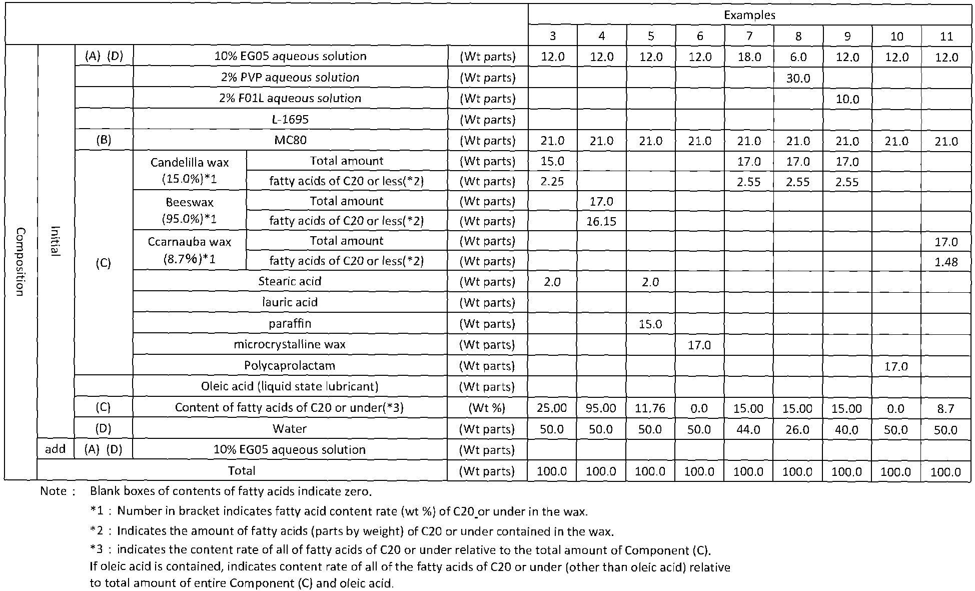

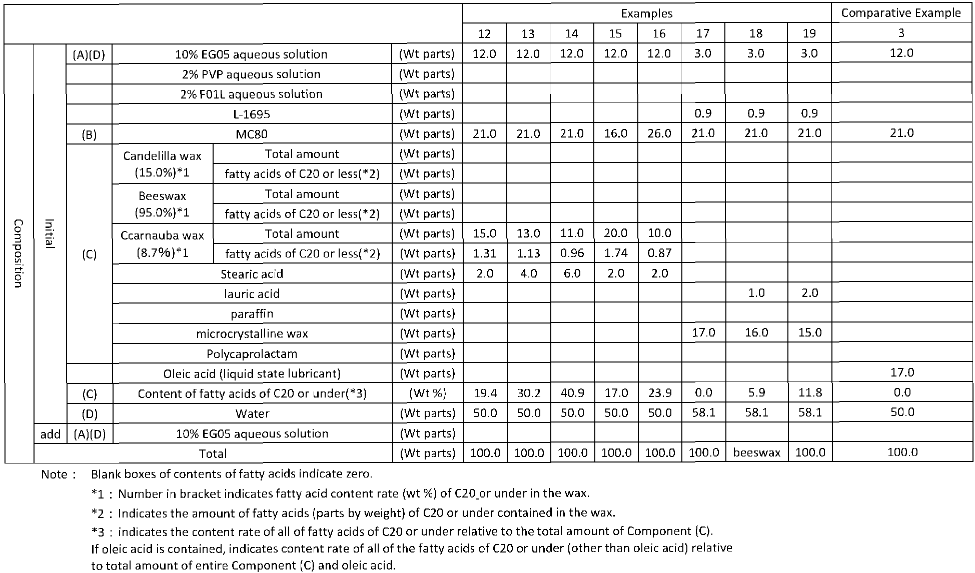

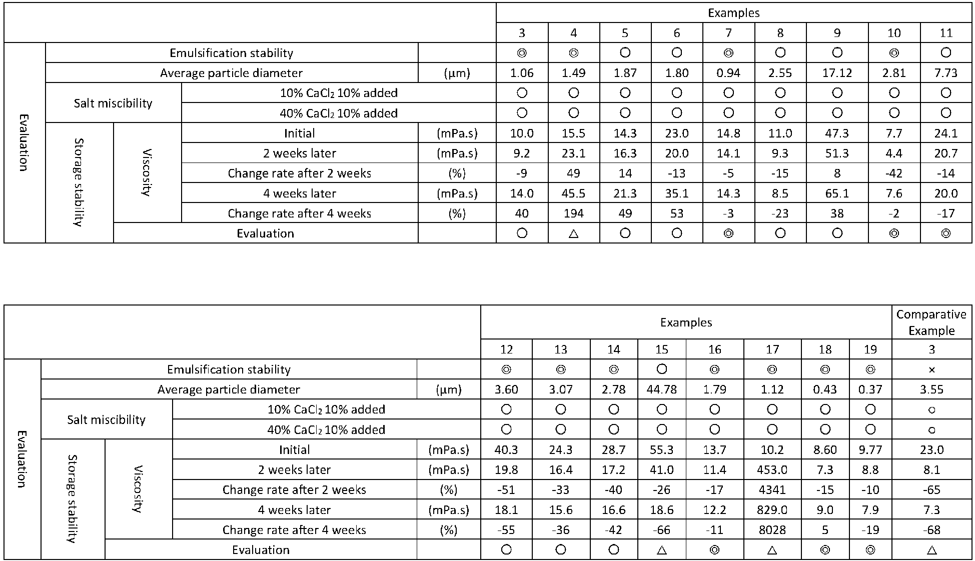

### (Results)

In Example 3, the solid oil was used in combination with stearic acid. Emulsification stability during production was improved, and when 10% of a 40% aqueous solution of calcium chloride (CaCl₂) was added, no agglomerates were produced and good salt miscibility was observed. while the viscosity change from the initial stage to 4 weeks later was 40%, Example 3 showed good storage stability because there is no loss of fluidity.

Example 4 uses beeswax, which is high in fatty acid content, as the solid oil. Emulsification stability was improved during production, and when 10% of a 40% aqueous solution of calcium chloride (CaCl₂) was added, no agglomerates were produced and good salt miscibility was exhibited. Although the viscosity increased between the initial stage and after 4 weeks by 194%, fluidity was not impaired.

Example 5 uses paraffin as the solid oil and also uses stearic acid in combination. When 10% of a 40% aqueous solution of calcium chloride (CaCl₂) was added, no agglomerates were produced and good salt miscibility was observed. The viscosity change between the initial stage and 4 weeks later was 49%, showing good storage stability with no loss of fluidity.

Example 6 uses microcrystalline wax as the solid oil. When 10% of a 40% aqueous solution of calcium chloride (CaCl₂) was added, no agglomerates were produced and good salt miscibility was exhibited. The rate of change in viscosity between the initial stage and four weeks later was 53%, showing good storage stability with no loss of fluidity.

In Example 7, the amount of the PVA of Example 1 was increased. Emulsification stability during production was improved, and when 10% of a 40% aqueous solution of calcium chloride (CaCl₂) was added, no agglomerates were produced and good salt miscibility was exhibited. The rate of change in viscosity from the initial stage to 4 weeks later was -3%, showing good storage stability due to the low rate of change in viscosity.

In Example 8, half of the PVA of Example 1 was changed to PVP. When 10% of a 40% aqueous solution of calcium chloride (CaCl₂) was added, no agglomerates were produced and good salt miscibility was observed. The rate of change in viscosity from the initial stage to 4 weeks later was -23%, showing good storage stability with no loss of fluidity.

In Example 9, to Example 1, nanofibers were further added by the amount 1/6 times the amount of the PVA. When 10% of a 40% aqueous solution of calcium chloride (CaCl₂) was added, no agglomerates were produced and good salt miscibility was exhibited. The viscosity change between the initial stage and after 4 weeks was 38%, showing good storage stability with no loss of fluidity.

In Example 10, polycaprolactone is used as the solid oil. A suspension with good emulsion stability during production was obtained, and when 10% of a 40% aqueous solution of calcium chloride (CaCl₂) was added, no agglomerates were produced and good salt miscibility was exhibited. While the viscosity change between the initial stage and after 2 weeks was -42%, showing a decreasing tendency, the viscosity change between initial stage and after 4 weeks was -2%, showing good storage stability with no loss of fluidity.

In Example 11, Carnauba wax with a high melting point was used as the solid oil. When 10% of a 40% aqueous solution of calcium chloride (CaCl₂) was added, no agglomerates were produced and good salt miscibility was observed. The viscosity change rate between the initial stage and 4 weeks later was -17%, indicating good storage stability due to the small rate of change in viscosity.

Example 12 is the solid oil combined with stearic acid. Emulsion stability during production improved, and when 10% of a 40% aqueous solution of calcium chloride (CaCl₂) was added, no agglomerates were produced and good salt miscibility was exhibited. While the viscosity change between the initial stage and after 4 weeks was -55%, there was no loss of fluidity, so that the storage stability was good.

In Example 13, the solid oil was used in combination with a larger amount of the stearic acid used in Example 12. Emulsion stability during production improved, and when 10% of a 40% aqueous solution of calcium chloride (CaCl₂) was added, no agglomerates were produced, so that good salt miscibility was observed. While the viscosity change rate from the initial stage to 4 weeks later was -36%, there was no loss of fluidity, so that the storage stability was good.

In Example 14, the solid oil was used in combination with a larger amount of the stearic acid used in Example 13. Emulsion stability during production improved, and when 10% of a 40% aqueous solution of calcium chloride (CaCl₂) was added, no agglomerates were produced and good salt miscibility was exhibited. While the viscosity change rate from the initial stage to 4 weeks later was -42%, there was no loss of fluidity, so that the storage stability was good.

In Example 15, the content ratio of the UV absorber was lowered and the content ratio of the solid oil was increased. The average particle size was 44.78 pm because the viscosity increased during emulsification due to the increased content ratio of the solid oil. However, when 10% of a 40% aqueous solution of calcium chloride (CaCl₂) was added, no agglomerates were produced and good salt miscibility was observed. While the viscosity change rate from the initial stage to 4 weeks later was -66%, there was no loss of fluidity, so that the storage stability was good.

In Example 16, the content ratio of the UV absorber was increased and the content of the solid oil was reduced. The average particle size was 1.79 pm because the viscosity during emulsification was reduced by lowering the content ratio of the solid oil. When 10% of a 40% aqueous solution of calcium chloride (CaCl₂) was added, no agglomerates were produced and good salt miscibility was observed. The viscosity change rate was -11% from the initial stage to 4 weeks later, indicating good storage stability due to the low rate of change in viscosity.

In Example 17, the amount of the 10% PVA aqueous solution of Example 6 was reduced by 25% and the active ingredient of the reduced amount was replaced with sucrose laurate. Regarding the emulsion stability during production, when 10% of a 40% aqueous solution of calcium chloride (CaCl₂) was added, salt miscibility was good with no agglomerates. Although the viscosity increased between the initial stage and 2 weeks later, at the rate of about 4000%, and between the initial stage and 4 weeks later, at the rate of about 8000%, fluidity was not impaired.

In Example 18, a portion of the microcrystalline wax of Example 17 was replaced with lauric acid. Regarding the emulsion stability during production, when 10% of a 40% aqueous solution of calcium chloride (CaCl₂) was added, salt miscibility was good with no agglomerates. The viscosity change rate was -15% from the initial stage to 2 weeks later and 5% from the initial stage to 4 weeks later, indicating good storage stability.

In Example 19, a portion of the microcrystalline wax in Example 18 was replaced with more lauric acid. Regarding the emulsion stability during production, when 10% of a 40% aqueous solution of calcium chloride (CaCl₂) was added, salt miscibility was good with no agglomerates. The viscosity change rate was -10% from the initial stage to 2 weeks later and -19% from the initial stage to 4 weeks later.

Comparative Example 3, on the other hand, used oleic acid, which is not a solid oil. It was found that the emulsion was in an emulsified state with oil floating, the emulsion stability was insufficient, and the UV absorber was not fully carried.

## Claims

1. A method for producing a composition containing (A) polyvinyl alcohol, (B) an oil-soluble UV absorber, (C) a solid oil, and (D) water, **characterized in that** said (B), (C) and (D) are emulsified in the presence of said (A) at a temperature above the melting point of said (C).

2. A method for producing a composition according to claim 1, **characterized in that** said composition is used in a UV protective cosmetic.
